# EUROPEAN PATENT SPECIFICATION

(11) **EP 3 474 996 B1**
(45) Date of publication and mention of the grant of the patent: **24.02.2021**
(21) Application number: 17734427.2
(22) Date of filing: 22.06.2017
(51) Int. Cl.: B05B 14/00, B05B 3/06, B05B 3/04, B05B 7/00, A61L 9/14, B01D 47/06, B05B 3/02

(54) **A MISTING APPARATUS AND METHOD OF USE**
BESCHLAGVORRICHTUNG UND VERFAHREN ZUR VERWENDUNG
APPAREIL DE PULVÉRISATION ET PROCÉDÉ D'UTILISATION

(30) Priority: 23.06.2016 GB 201610957
(43) Date of publication of application: 01.05.2019
(73) Proprietor: Brendon Limited, Taunton, Somerset TA4 2TU (GB)
(72) Inventor: HENDY, Robert John, Milverton Somerset TA4 1LR (GB)
(74) Representative: Haseltine Lake Kempner LLP
(86) International application number: PCT/GB2017/051830
(87) International publication number: WO 2017/221017

(56) References cited:
- WO-A1-2015/010160
- WO-A1-2015/101227
- CN-U- 203 695 297
- US-A- 3 387 432

## Description

The invention relates to a misting apparatus for producing mist from a pressurized liquid, such as water.

Dust is a potential hazard to personnel, for example at construction sites and industrial facilities. It is known to provide dust suppression units which eject a plume of water droplets, or mist, in order to take dust out of suspension in the air. The physical mechanism is related to the relative size between the droplets in the mist and the dust particles. Similar technology is used, typically with smaller droplets, for odour control (for example, at sewage treatment works and other facilities that suffer from odours).

Known dust or odour suppression units have an electrically-powered fan and a ring of water-injecting nozzles to emit a spray. Such units are typically powered by relatively high voltage AC electrical power, for example 415 volt 3-phase power.

Known dust or odour suppression, such as disclosed in WO 2015/010160, are typically used on large sites where there may be a regulatory requirement to do so. As such, they are typically bulky, may require professional installation and safety monitoring, and may therefore be expensive to operate.

According to a first aspect of the invention there is provided a misting apparatus according to claim 1. Said misting apparatus is for producing a mist from a pressurized liquid, comprising: a fan shaft having a rotational axis; a rotary jet assembly configured to drive the fan shaft and comprising a radially extending arm configured to convey the pressurized liquid to a jet nozzle, wherein the jet nozzle is configured to eject the pressurized liquid in a jet to generate a jet reaction force having a tangential component; a fan coupled to the fan shaft for driving an airflow; and a spray nozzle configured to eject the pressurized liquid into the airflow to produce a mist.

There may be a plurality of radial arms, for example two diametrically opposed arms. The fan shaft may be integral with the rotary jet assembly. The fan shaft or hub of the rotary jet assembly may comprise a fluid pathway for providing the liquid to the rotary jet assembly. The fan shaft may comprise a fluid pathway for providing the liquid to the spray nozzle.

The jet assembly may comprise a manifold configured to receive the pressurized liquid. The manifold may comprise: a first outlet configured to provide the liquid to a conduit extending along the radial arm to the jet nozzle; and a second outlet comprising the spray nozzle or configured to provide liquid to the spray nozzle. Accordingly, the manifold rotates in use. A hub and/or shaft of the jet assembly may comprise the manifold.

The fan shaft may be coupled to a mount by a rotary seal. The mount may provide pressurized liquid to the fan shaft through the rotary seal. The fan shaft may be coupled indirectly, for example via a hub of the rotary jet assembly.

The misting apparatus may be for receiving a pressurized liquid of variable pressure. The jet nozzle and the spray nozzle may be fluidically coupled so that the flow rate of liquid through the jet nozzle is approximately proportional to the flow rate of liquid through the spray nozzle over a pressure range. For example, the flow rates may be substantially proportional over a range of approximately 50-200bar.

The spray nozzle may be mounted to rotate together with the fan shaft in use. For example, the spray nozzle may be directly mounted on the fan shaft, or on a component fixed to rotate with the fan shaft, or may be integral with the fan shaft. The spray nozzle may be detachably attached to the fan shaft.

The fan may comprise a plurality of blades each having a radially outer tip. The jet nozzle may be disposed radially outward of the tips of the blades.

The misting apparatus may further comprise a housing. The housing may comprise a jet containment disposed around the rotary jet assembly for capturing liquid ejected from the jet nozzle. The misting apparatus may comprise a collector for collecting liquid captured by the jet containment for recirculation to a pump or reservoir. The jet containment may be configured so that liquid captured therein flows under the action of gravity to the collector.

The misting apparatus may further comprise a drive unit coupled to the fan shaft so as to be driven to rotate by rotation of the fan shaft. The drive unit may be for driving a liquid return pump for pumping collected liquid from the collector. The misting apparatus may comprise an attachment point for detachably attaching a liquid return pump so that in use a liquid return pump attached to the attachment point is driven by the drive unit. For example, the attachment point may be configured to engage with the liquid return pump by a quick release or snap-fit mechanism. The attachment point may be formed in the collector or another part of the housing. The attachment point may comprise an opening for insertion of a shaft of the liquid return pump to engage with the drive unit. A shaft of the drive unit may protrude through or adjacent to the attachment point for engaging with the liquid return pump. The misting apparatus may further comprise a liquid return pump coupled with the drive unit and arranged to pump liquid from the collector.

The jet containment may have a radially inner opening through which the radial arm of the rotary jet assembly extends. The jet containment may have an axial extent which is greater than the axial extent of the opening so that liquid captured in the jet containment can flow along a radially inner wall of the jet containment axially adjacent the opening. The jet containment may comprise a radially outer wall to contain liquid ejected from the jet nozzle and a radially inner wall to inhibit ejected liquid from entraining in the airflow driven by the fan.

The radial arm may extend past the radially inner wall of the jet containment so that the jet nozzle is radially outward of the radially inner wall. The lip of the radially inner wall axially adjacent the radial arm may be provided with a flange to inhibit ejected liquid from entraining in the airflow driven by the fan. The jet containment may comprise a forward portion forward of the radially inner opening and bounded by a forward radially inner wall and/or an aft portion aft of the radially inner opening and bounded by an aft radially inner wall.

The housing may comprise an airflow nozzle downstream of the jet containment, and the jet containment may define a channel radially outward of the airflow nozzle for capturing liquid ejected from the jet nozzle. The jet nozzle may be radially outward of the airflow nozzle.

The housing may comprise an airflow intake upstream of the jet containment, and the jet containment may define a channel radially outward of a junction between the airflow intake and the jet containment. The junction may be defined where an axially extending portion of the airflow intake and a radially extending portion of the jet containment meet. The jet nozzle may be radially outward of the junction between the airflow intake and the jet containment.

The jet containment may define a substantially annular channel.

The jet nozzle may be configured to eject the pressurized liquid along a direction having an axial component. Accordingly, when the misting apparatus comprises a jet containment, the jet may be directed axially away from a radially inner opening in the jet containment that is provided for the radial arm. This may prevent ejected liquid from being entrained in the airflow.

The apparatus may comprise a housing including an airflow nozzle, and the apparatus may further comprise a drip collector coupled to a downstream end of the airflow nozzle, the drip collector having a radially inner wall defining an outlet of the apparatus radially within the downstream end of the airflow nozzle, a radially outer wall disposed around the downstream end of the airflow nozzle, and a channel therebetween for collecting drips conveyed along the wall of the airflow nozzle. The drip collector may have a plurality of holes in the radially inner wall. Such holes may prevent buffeting.

The misting apparatus may further comprise a composite line for delivery of pressurized liquid from a source of pressurized liquid and for return of liquid captured from the jet nozzle to the source of pressurized liquid. The composite line may be flexible.

According to a second aspect there is provided an apparatus, comprising: a portable device for pressurizing liquid; a misting apparatus in accordance with the first aspect fluidically coupled to the portable device to receive pressurized liquid. The misting apparatus may be mounted above the portable device on a support. The support may be a post, such as a telescopic post.

There may be a plurality of misting apparatus in accordance the first aspect each fluidically coupled to the portable device to receive pressurized liquid. The portable device may be configured to pressurize liquid to between 30bar-200bar. The portable device may be configured to provide pressurized liquid at a flow rate of between 5-60 litres per minute.

The liquid may be water.

According to a third aspect there is provided a method of suppressing dust in a hazardous environment or controlling odour according to claim 15, the method comprising: providing an apparatus in accordance with the first aspect with pressurized liquid between 30bar-200bar at a flow rate of between 5-60 litres per minute to eject a mist to suppress dust or control odour.

The invention may comprise any combination of the features and/or limitations referred to herein, within the scope of the claims and except combinations of such features as are mutually exclusive.

The invention will now be described, by way of example only, with respect to the accompanying drawings, in which:
Figure 1 schematically shows a side view of a portable device for pressurizing a liquid with a misting device mounted on a post;
Figure 2 schematically shows in perspective view an exterior of the misting device of Figure 1;
Figure 3 schematically shows a perspective view of inner components of the misting device of Figure 1;
Figure 4 schematically shows a side view of the inner components of Figure 3;
Figure 5 schematically shows a cross-sectional side view of the rotary jet assembly and housing of the misting device of Figure 1;
Figure 6 schematically shows a perspective exploded view of the misting device of Figure 1;
Figure 7 schematically shows a perspective view of a return pump arrangement for the misting apparatus of Figure 1; and
Figure 8 schematically shows a portable device for pressurizing a liquid with a plurality of misting devices coupled by flexible conduits.

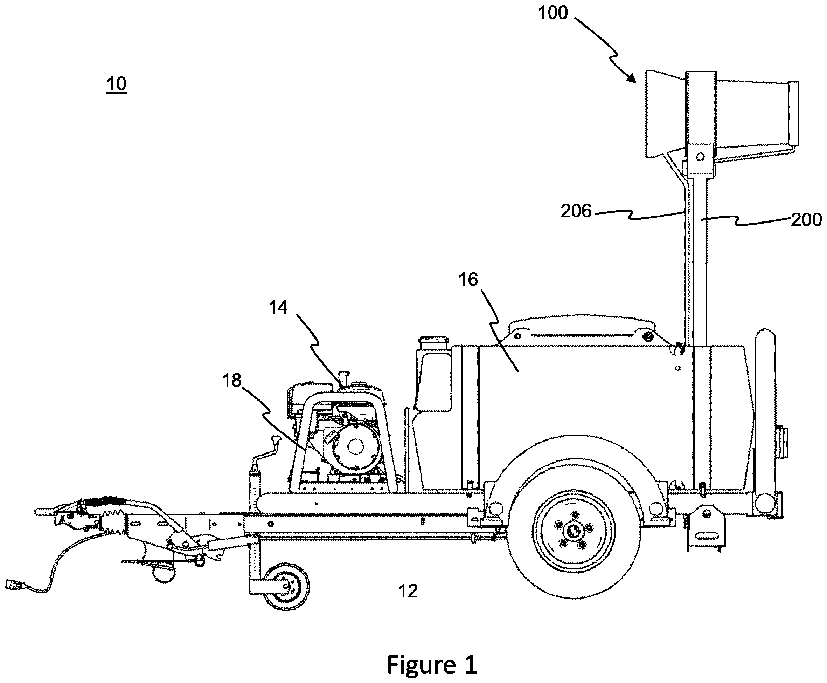

**Figure 1** shows a portable device 10 for pressurizing a liquid, in particular water, and a misting apparatus 100. The device 10 is a portable pressure unit typically used for pressure washing, and in this particular example comprises a wheeled trailer 12 supporting a generator 14, water reservoir 16 and pump 18.

A misting apparatus 100 is provided mounted on a support post 200 to eject a mist of water droplets from an elevated position. In this example, there is a supply line 206 for supplying pressurized water from the portable device 10 to the misting apparatus 100, and a support post 200 acts as a return line for recirculating water, as will be described below.

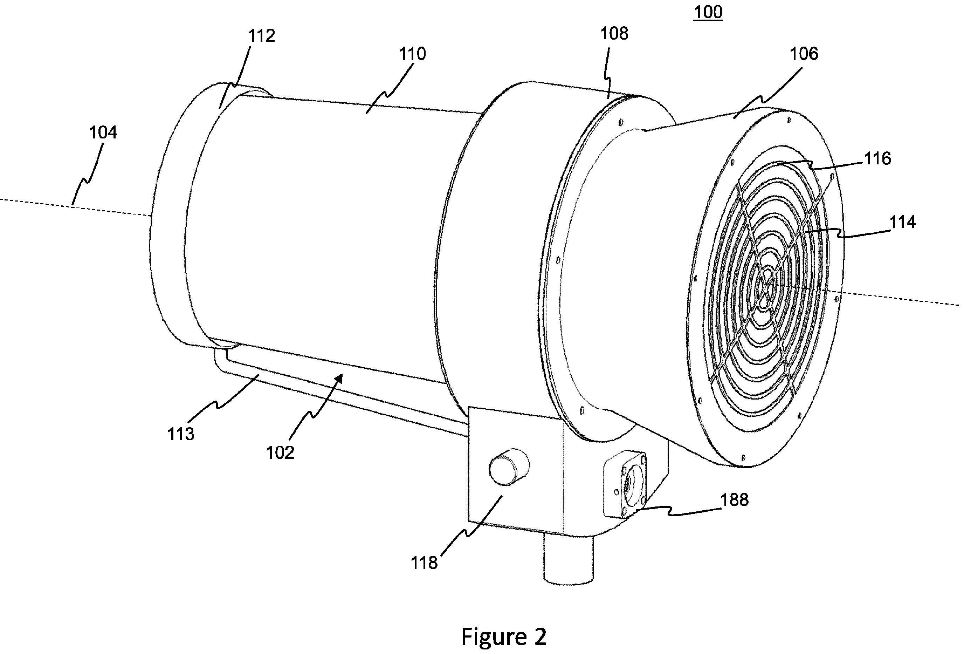

**Figure 2** shows exterior components of the misting apparatus 100 in further detail. The misting apparatus 100 includes a housing 102 extending along a central axis 104 of the apparatus 100 and comprising an air intake 106, a jet containment 108 and an airflow nozzle 110 provided with a drip collector 112. The air intake 106 is for receiving air from the surrounding environment. It comprises an aft opening 114 fitted with a safety grate 116, and a substantially conical body tapering forwardly to accelerate the airflow therethrough.

The jet containment 108 is disposed downstream of the air intake 106 and extends radially outward from a circular junction between the air intake 106 and the jet containment 108 to define an annular channel disposed radially outwardly of an air flowpath through the misting apparatus 100, as will be described in detail below.

The airflow nozzle 104 is downstream of the jet containment 108 and comprises a frustoconical tapering body at a taper angle of approximately 15º downstream. In this example apparatus, the air intake 106, jet containment 108 and airflow nozzle 104 are generally axisymmetric and coaxial with one another.

Below the jet containment 108 there is a collector 118 for collecting water captured within the jet containment 108. The collector is generally cuboidal with curved upper edges to couple to a lower portion of the jet containment 108 having openings for water to flow through. In this example the collector 118 is a separate component from the jet containment 108, which aids in servicing and maintenance, but in other examples may be integrally formed with the jet containment.

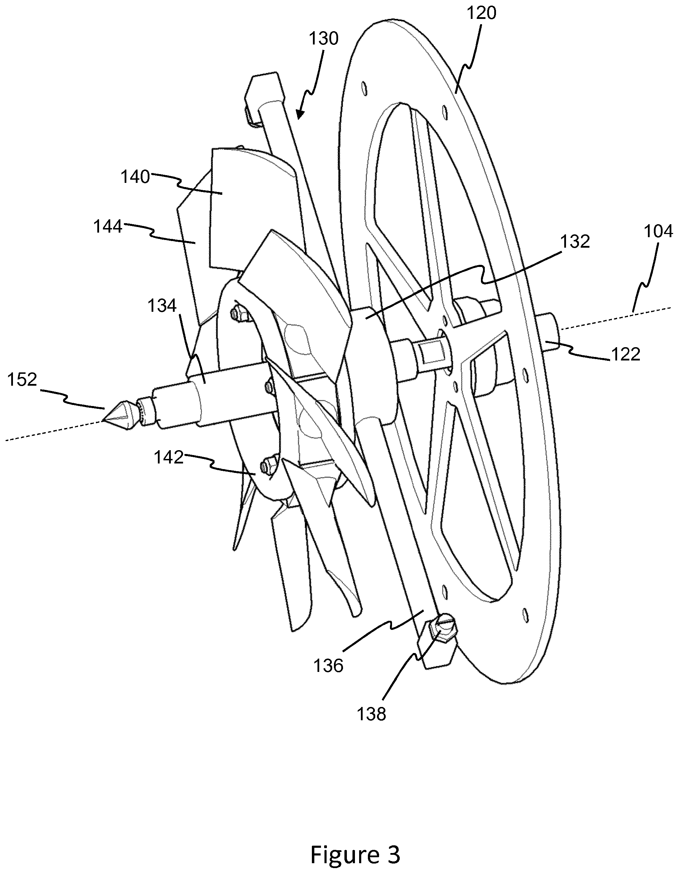

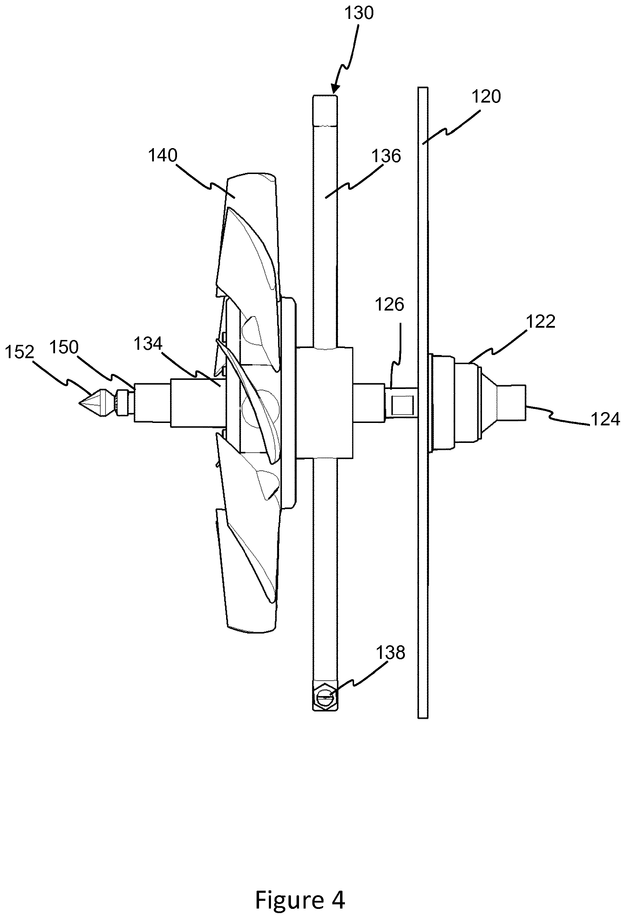

**Figures 3** **and** **4** show internal components of the misting apparatus 100 located within the housing 102. A circular mounting ring 120 comprising a central hub, radially outer rim and supporting spokes is configured to be installed within the housing against an aft radially-extending annular wall of the jet containment, for example by bolts extending through the jet containment 108 and through the mounting ring 120. When assembled in the housing 102, the mounting ring 120 has a central opening which is coaxial with the axis 104 of the misting apparatus, which is therefore defined as an axis of rotation for rotational components of the misting apparatus, as will be described in detail below.

A rotary seal 122 having an aft stationary part and a forward rotating part is mounted to the mounting ring 120 upstream of the mounting ring, for example by a series of bolts or rivets that extend through a flange of the rotary seal 122 and the hub of the mounting ring 120. The rotary seal 122 is configured to receive a high pressure liquid in an axial inlet 124 of the stationary part, for example water at up to 300bar, and to discharge the high pressure liquid through a rotating outlet of the rotating part. In this example, the rotary seal 122 is configured so that the outlet is coaxial with the rotational axis 104 of the mounting assembly. In this example, the rotating part is in the form of a rotatable seal shaft 126 that extends through the central opening of the mounting ring 102.

A rotary jet assembly 130 is mounted on the seal shaft 126 downstream of the mounting ring 120 so as to rotate together with the seal shaft 126 and receive pressurized liquid therefrom. In this example, the jet assembly 130 comprises a hub 132 mounted on the seal shaft 126, a fan shaft 134 extending from the hub 132, and two radially extending arms 136 extending from the hub 132. In this particular example, the hub 132 and fan shaft 134 are integrally formed, but in other examples they may be distinct components coupled to rotate together.

In this example the two radial arms 136 are diametrically opposed from one another, such that they extend along the same diametrical and radial line through the hub 132. Each radial arm 136 has a radial extent such that its distal end extends into the annular channel defined by the jet containment 108. Each radial arm 136 is provided with a jet nozzle 138 configured to eject a jet of pressurized liquid along a direction so as to generate a jet reaction force on the jet assembly 130 having a tangential component. In this particular example, each radial arm defines a fluid pathway for conveying fluid from a manifold within the jet assembly (in particular, within the hub 132 and shaft 134) to the respective jet nozzle 138. As shown in Figures 3 and 4 each jet nozzle 138 extends substantially tangentially with respect to the rotational axis 104, but in other examples, the or each jet nozzle may extend along a direction inclined with respect to the tangential direction, as will be described in detail below. It will be appreciated that in other examples, there may be a single radial arm 136, or more than two radial arms 136.

The fan shaft 134 extends along the rotational axis 104 forwardly of the hub 132. A fan 140 is mounted on the fan shaft 134 so as to rotate together with the fan shaft 134. In this example, the fan 140 comprises a fan hub 142 having a central opening for locating over the fan shaft 134, for example by bolts extending through axial holes in the hub and arranged to be received in a flange of the fan shaft 134. The fan 140 further comprises a plurality of fan blades 144 radially extending from the hub 142 and configured to rotate to propel airflow through the misting apparatus in a direction from the air intake 106 to the airflow nozzle 110.

The fan 140 has an axial location downstream of the hub 132 of the rotary jet assembly 130, and also downstream of the jet containment 108 and within the airflow nozzle 110. In this example the fan blades 144 have a radial extent which is less than the radial extent of the radial arms 136 and the radial position of the jet nozzles 138 of the jet assembly 130. For example, the radius of the tips of the fan blades 144 may be approximately 15cms, whereas the radial position of the jet nozzles 138 may be approximately 20cms.

The fan shaft 134 extends through and axially forward of the fan 140 and in this example terminates at a nozzle connection 150 at the extreme axial end of the fan shaft, coaxial with the rotational axis. The nozzle connection 150 is configured to receive a spray nozzle 152, which in this example is detachably attachable to the nozzle connection 150, for example by a screw thread or other fastener. The spray nozzle 152 therefore rotates together with the fan shaft. In this particular example, the spray nozzle 152 has a plurality of angularly spaced holes configured to eject respective jets of liquid substantially axially towards a radially outwardly extending conical surface of the nozzle 152, such that the jets impinge on the conical surface and are dispersed radially outwardly into an airflow through the fan 140 to form droplets of a mist, as will be described in detail below. The fan shaft 134 defines an internal fluid pathway which fluidically couples the nozzle connection 150, and thereby the spray nozzle 152 received therein, with the manifold within the jet assembly 130.

The manifold may be considered to comprise the fluid pathways through the or each rotary arm to the jet nozzles and the fluid pathway through the fan shaft 134 to the nozzle connection and nozzle. The manifold is defined within the rotary jet assembly and is thereby configured to rotate together with the radial arms 136, jet nozzles 138 and the fan 140. In particular, the manifold comprises an inlet to receive pressurized liquid (in this example water from the outlet of the seal shaft 126), and outlets for the or each jet nozzle 138 and the spray nozzle 152. Each outlet may be considered the outlet to the individual fluid pathways extending through the radial arm, or the outlet of such fluid pathways at the respective nozzles 138, 152.

In this example, the manifold has no control valves or other valves between the jet nozzles 138 and the spray nozzle 152, such that the amount of flow through each nozzle (and fluid pathway thereto) depends on the pressure drop through the respective nozzle and supply route, as will be described in detail below with respect to a method of use.

In other examples, there may be a spray nozzle formed in the fan shaft (rather than a detachably attachable spray nozzle), for example one or more holes formed in the fan shaft 134. Such holes may be radially extending in a side (e.g. a cylindrical side) of the fan shaft 134, and/or may be formed in an axial end face of the fan shaft 134.

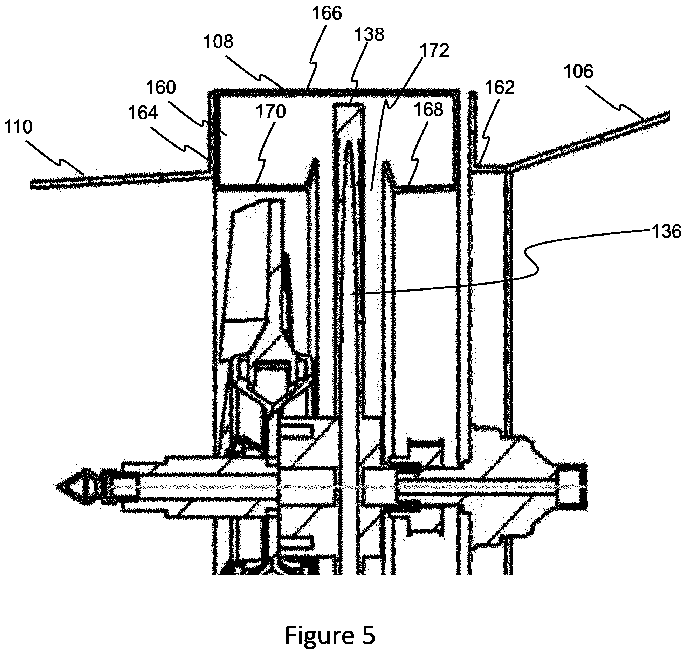

**Figure 5** shows a partial cross-sectional view of a radial arm 136 and jet nozzle 138 extending into the jet containment 108. As described above, the jet containment 108 defines an annular channel 160 that in this example extends radially outwardly of a junction 162 between the aft air intake 106 and the jet containment 108, and also radially outwardly of the proximal end (i.e. the end towards the middle of the apparatus as a whole) of the airflow nozzle 110. Figure 5 shows further features of this arrangement. In particular, the jet containment has aft and forward radially extending walls 164 coupled by a radially outer wall 166, and also two radially inner walls: an aft radially inner wall 168 and a forward radially inner wall 170. The radially inner walls define therebetween an opening 172 through which the radial arm 136 extends. The radially inner walls are provided with flanges adjacent the opening to retain captured water on the radially inner wall. In other examples, there may only be one radially inner wall, and the opening may be axially adjacent the respective wall.

The jet containment 108 is configured to capture liquid ejected by the jet nozzles 138. n particular, the radially outer wall 166 is configured to retain the liquid by limiting the radial extent to which it can be ejected, and the or each radially inner wall 168, 170 is configured to prevent such liquid from falling or rebounding radially inwardly towards the central airflow extending through the misting apparatus (i.e. towards the rotational axis 104). It will be appreciated that in use liquid that is ejected axially forward and/or aft of the opening 172 will either fall directly to the lower side of the annular channel under gravity, or shall fall onto a radially inner wall 168, 170 and flow thereabout towards the lower side of the annular channel 160. In some examples, the jet nozzles 138 may be configured to eject liquid along a direction inclined slightly out of a plane normal to the rotational axis so as to have an axial extent, so as to cause more of the liquid to be ejected over a radially inner wall 168, 170 rather than the opening 172. In one such example, such jets of a jet nozzle 138 may oppose one another with respect to such a normal plane, such that there is substantially equal flow forward and aft, and any axial reaction force from the jets is thereby substantially equalised.

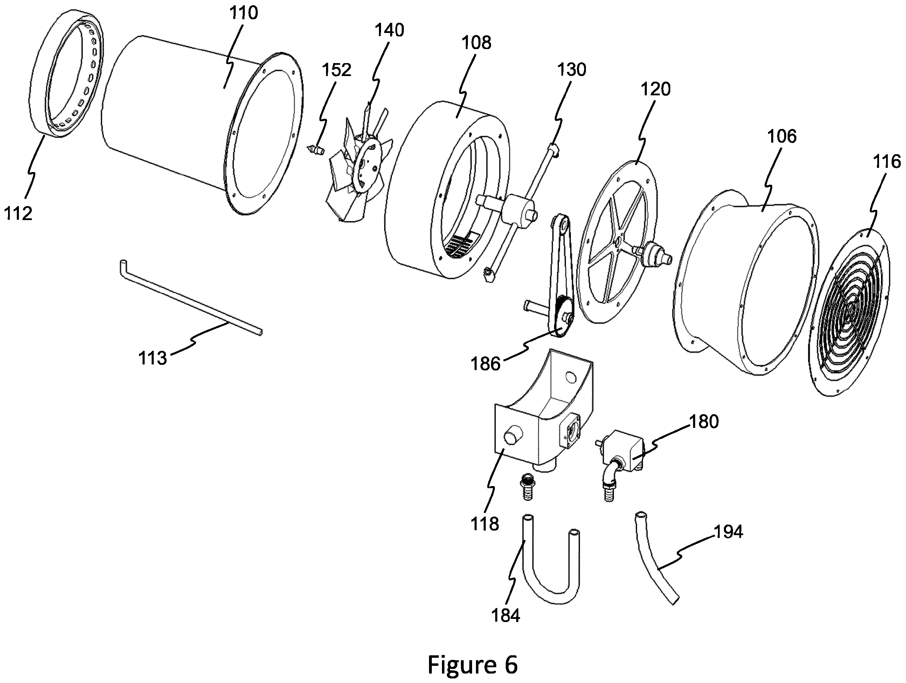

**Figure 6** shows an exploded view of components of the misting apparatus 100. Components not previously described in detail include the drip collector 112 and a return pump 180.

The drip collector 112 is in the form of an annular C-shaped channel having a radially outer wall, a radially inner wall, and a downstream curved end connecting the two. The radially outer wall is configured to fit over the distal (i.e. farthest) end of the airflow nozzle 110, whereas the radially inner wall is configured to fit within the distal opening of the airflow nozzle 110 and be slightly separated therefrom such that drips collecting on and flowing axially down the inner wall of the airflow nozzle 110 flow directly into the C-shaped channel. Under gravity and in the absence of a throughflow of air, such drips shall flow circumferentially around the C-shaped channel to a lower point of the channel (i.e. a point closest to the ground level when positioned above ground). A drip collection hose 113 is provided to collect such drips and, under gravity, deliver them to the collector 118 (via an opening in the collector 118). The collection hose 113 may be configured to be inclined so that such drips are reliably returned to the collector, for example the hose may be flexible but appropriately sized to avoid bunching, or may be substantially rigid or relatively stiff to prevent it becoming bent out of the desired inclined configuration.

The drip collector may have a plurality of holes in the radially inner wall. Such holes may prevent buffeting. In other examples, there may be no such holes.

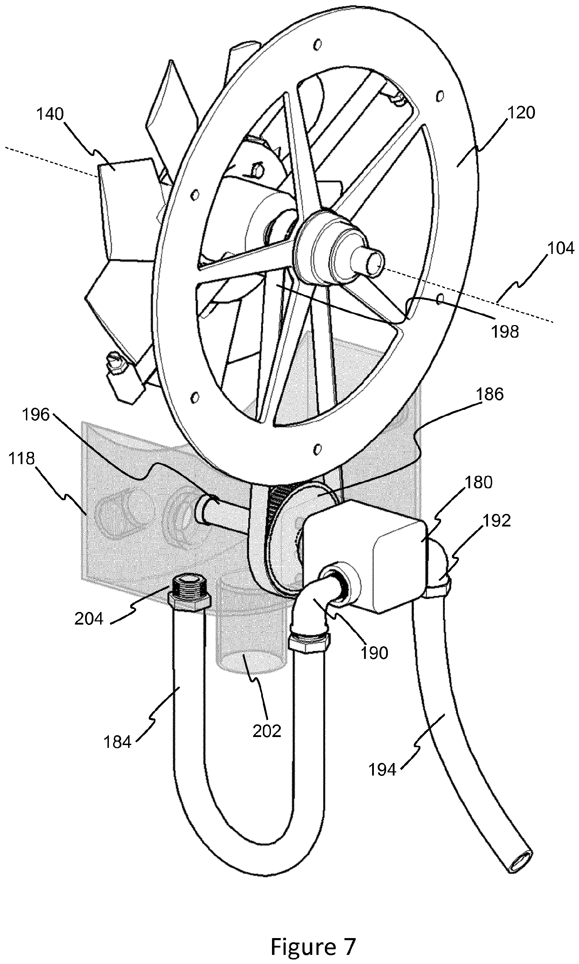

**Figure 6** **and** **7** show the collector 118 and a return pump 180 of the misting apparatus, with the misting apparatus coupled to a supporting return conduit 200.

In this example, the return conduit 200 functions as a support post for the misting apparatus, as shown in Figure 1. In other examples, the return conduit 200 may be flexible.

In this example, the misting apparatus 100 is coupled to the supporting return conduit 200 by a flexible joint (not shown). For example the flexible joint may include an adjustable hinge so that an operator can set the rotational axis of the misting apparatus within a range of attitudes (pitches). In other examples, the flexible joint may include an oscillating mechanism for rotating the misting apparatus around a vertical axis.

In this example, the return conduit 200 serves as a return line for returning liquid captured by the jet containment 108 and collector 118 to the source of pressurized liquid, such as the portable device 10 described above with respect to Figure 1. Further, a liquid return pump 180 and associated return lines are provided for recirculating the collected water under power, as will be described in detail below.

As shown in **Figure 7****,** the collector 118 is provided with a main outlet 202 which can be inserted into a support post or the return conduit 200 (which may be a supporting return conduit, as described above), or a flexible joint arrangement arranged therebetween. The collector 118 is also provided with a secondary outlet 204 adjacent the main outlet 202. The secondary outlet 204 may be provided with a quick-release attachment for attaching a further or alternative return line.

The return pump 180 is coupled to the collector at an attachment point 188 (shown in Figure 2), and has an inlet 190 coupled to a primary return line 184 extending between the collector 118 and the return pump 180, and an outlet 192 coupled to a secondary return line 194 extending between the return pump 180 to the portable device 12.

As shown in Figure 7, a drive unit 186 for the return pump is provided within the housing 102. In this example, the drive unit 186 is sealed within a partitioned portion of the collector 118 to protect the drive unit 186 from water collected in the collector 118. The drive unit 186 comprises a wheel (or gear) mounted on a drive shaft 196 within the housing and substantially parallel with (and offset from) the rotational axis 104. The drive unit 186 is coupled to the seal shaft by a drive belt 198, such that the drive unit 186 is driven to rotate as the seal shaft is driven to rotate (by virtue of being coupled to the rotary jet assembly 130).

The return pump 180 comprises a stub shaft (not shown) receivable (and in this example, received) in the attachment point 188 so as to be driven by rotation of the drive unit 186 (e.g. a keyed shaft). The return pump 180 further comprises an impeller for driving liquid received through the inlet 190 from the primary return line 184 and out through the outlet 192 to the secondary return line 194.

In some examples, the return pump 180 and attachment point 188 may be configured to cooperate with each other, for example by a quick release or snap-fit mechanism. For example, the return pump 180 could be coupled to the attachment point 188 by a push-fit mechanism, and secured by a removable pin engaging through walls slots in both the return pump 180 and the attachment point 188. Accordingly, the return pump 180 can be quickly connected to the misting apparatus 100, and the return line routed through the return pump 180, in order to drive the collected liquid through the return line under power (rather than relying on gravity alone).

Accordingly, the operator may connect the misting apparatus for liquid return in a variety of different ways. In a first example, as described above, the main outlet 202 may be received on a supporting return line 200 and may be open to return collected water through the supporting return line 200 by gravity. This arrangement may be supplemented by additionally connecting the liquid pump 180 and corresponding return lines 184, 194 to return the collected water under power. In other examples, the main outlet 202 may be blocked off, for example with a plug, and liquid return may be solely via the secondary outlet 204. In such examples, the main outlet 202 may still be used to provide a supporting attachment for the misting apparatus 100. The secondary outlet 204 may be provided with a quick release valve which opens when a return line is coupled thereto, and closes when the return line is removed.

In yet a further example, the misting apparatus may be provided with a mobile reservoir, for example a relatively small reservoir remote from the source of pressurized water. The misting apparatus may be supported on the mobile reservoir by the main outlet 202 forming a spigot connection with an inlet of the mobile reservoir, and the main outlet 202 may be open so that collected water drains into the mobile reservoir. The return pump 180 may be attached to the drive unit 186, and a return line 184 may extend between the mobile reservoir and the return pump 180, to direct collected water from the reservoir back to the source of pressurized water. The outlet of the reservoir may be configured so that a buffer supply of water is maintained in the reservoir, which may be useful to provide a base weight for the misting apparatus (i.e. to prevent movement or toppling of the misting apparatus). The buffer supply may also improve operation of the pump, since it may not be subject to intermittent lack of collected water.

An example of using the misting apparatus 100 will now be described, by way of example only.

In this particular example, the misting apparatus 100 is provided to suppress dust. An operator selects an appropriate spray nozzle 152 for dust suppression, for example based on the size of holes in the nozzles, and a supply pressure for the pressurized liquid (which in this example is water). In this example, pressurized water is supplied from the portable device 10 at a pressure of approximately 50bar. The spray nozzle 152 is selected so that, at a flow rate of approximately 30 litres per minute (through the misting apparatus 100 as a whole), approximately 10 litres per minute flows through the spray nozzle 152 to produce a mist in an airflow through the nozzle having a droplet size in the order of 50µm to 200µm, for example approximately 100 µm.

It will be appreciated that a spray nozzle 152 may provide a mist suitable for dust suppression (or odour control) over a range of pressures and flow rates, for example from 50bar to 200bar and flow rates of between 10 litres per minute to 40 litres per minute (through the misting apparatus as a whole).

Further, in certain configurations where a spray nozzle having particularly large or particularly small jet holes are required (for example, to achieve a desired droplet size), the jet nozzles may be replaced in order to achieve a desired flow rate through the spray nozzle and jet nozzles respectively.

In this example the misting apparatus 100 is coupled on the supporting return conduit 200 coupled to the portable device, such that the misting apparatus 100 is disposed above the portable device, for example at a height of approximately 2m above ground level.

The pressurized water is delivered through a supply line 206 extending between the reservoir 16 of the portable device and a port in the housing of the misting apparatus, with an internal supply line extending from the port on the interior of the housing to the rotary seal 122, from where it is transferred into the hub 132 of the rotary jet assembly. Within the manifold of the rotary jet assembly, the flow of pressurized water divides so that a first portion of the flow is conveyed along the radial arms 136 to the jet nozzles 138, and a second portion of the flow is conveyed through the fan shaft 134 to the nozzle attachment 150 and spray nozzle 152. The relative proportions of the first and second portions are determined according to the pressure drop through each flow path, and in this particular example approximately two thirds of the flow is conveyed along the radial arms 136 to the jet nozzles 138, whereas the remaining third is conveyed along the fan shaft 134 to the spray nozzle 152.

The jet nozzles 138 eject the pressurized water along a substantially tangential direction relative the rotational axis 104 into the annular channel 160 of the jet containment, thereby imparting a jet reaction force having an opposing tangential component on the radial arms 136. This jet reaction force causes the rotary jet assembly to rotate. In this particular example, the jet assembly 130 is caused to rotate at a velocity of approximately 2000rpm, but in other examples the jet assembly may rotate faster or slower, for example between 1000 and 3000rpm.

Rotation of the jet assembly 130, including the integral fan shaft 134, causes corresponding rotation of the fan 140 mounted thereto. The fan blades 144 of the fan 140 therefore rotate to propel an airflow through the mounting assembly. In particular, the fan blades 144 drive an airflow through the mounting assembly 140 in a direction substantially along the rotational axis 104 from upstream (outside of) the air intake 106, through the jet containment 108 radially within (i.e. inward of) the annular channel 160, through the airflow nozzle 110 and drip collector 112 to be discharged in a plume. For example, with the fan rotating at approximately 3000rpm the airflow may be discharged from the airflow nozzle 110 at a velocity of approximately 10m/s. In other examples, this exit velocity may be higher or lower, for example between 5m/s and 20m/s.

The remaining second portion of the pressurised water from the manifold within the rotary jet assembly 130 is conveyed along the fan shaft to be discharged from the spray nozzle 152. As described above, in this example the spray nozzle 152 is configured to discharge jets of pressurized water radially into the airflow through the airflow nozzle 110 to result in a mist having droplets of approximately 100µm.

The mist is discharged into the surrounding environment to suppress dust or odour. For example, dust or odour particles having a size of the same order of magnitude as the size of the droplets within the mist may be entrained together with the droplets and taken out of suspension in the air of the environment.

In order to adjust the speed of the airflow through the misting apparatus, and correspondingly adjust the quantity of water ejected through the spray nozzle, the operator may adjust the supply pressure and/or flow rate on the portable device 10 (or other source of pressurized liquid). For example, the operator may adjust a valve, such as a needle valve, on the pump 18 of the portable device 10.

As described above, the jet containment 108 is configured to capture and convey water discharged from the jet nozzles. In this particular example, approximately 80% of the water ejected through the jet nozzles 138 is captured by the jet containment 108 and collected in the collector 118 (i.e. through openings in a lower portion of the jet containment 108 adjacent the collector 118). The remaining 20% is considered to return through the radial opening 172 of the annular channel 160 of the jet containment 108, and is therefore entrained into the airflow through the misting apparatus 100.

Whilst in some examples the water collected in the collector may flow from the collector 118 back to a reservoir of the portable device 10 under gravity alone, in this example a return pump 180 is additionally provided between the collector 118 and portable device 10 in order to return the water under power, as described above.

The portable device and misting apparatus therefore conveniently provide the ability to generate a mist, for example for dust suppression or odour control. In particular, since the misting apparatus is powered by the pressurized liquid alone, there are no electronic components, or electricity supply to the misting apparatus. This may avoid the potential hazard of electrical equipment being exposed to water.

Further, the rotary jet assembly provides a particularly simple and inexpensive means for providing a rotational drive for the fan. The rotary jet assembly enables easy re-capture of the ejected liquid. The manifold of the rotary jet assembly provides a particularly simple and inexpensive means for directing the pressurized liquid to the jet nozzles (for rotary drive) and to the spray nozzle, and enables the relative proportions of these flows to be adjusted by switching nozzles, thereby providing simple and inexpensive control of the amount of liquid in a mist, and the rotary speed and therefore the velocity of the airflow through the mist. Further, providing the manifold and fluid pathways within the rotary jet assembly may result in an arrangement requiring few parts, and mounting the spray nozzle on the rotary shaft enables an even distribution of droplets in the airflow, without providing multiple misting nozzles.

The particular arrangement of the misting apparatus, as claimed, may result in a relatively inexpensive construction which may be suitable for use with relatively inexpensive and/or portable sources of pressurized liquid. For example, the misting apparatus may be coupled to sources of pressurized liquid typically used for pressure washers, and may therefore be particularly convenient for use in environments where there is a demand for dust suppression or odour control over a relatively limited area, or where there is demand for a low-cost, temporary, portable or adaptable system.

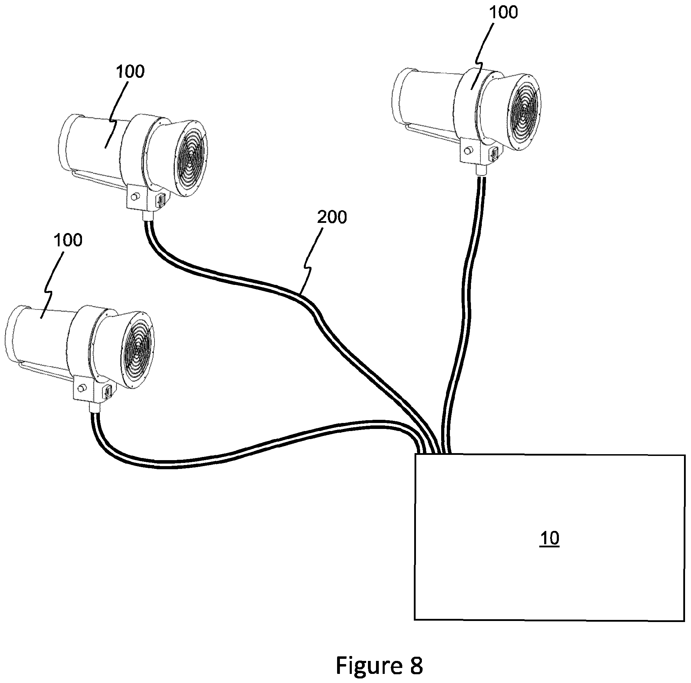

**Figure 8** shows an alternative implementation of the misting apparatus 100. In this particular example, a portable device 10 is provided to power multiple misting apparatus 100. As shown in Figure 8, three misting apparatus 100 are individually coupled via flexible supply conduits 200 to the same portable device 10, which has three separate outlets for pressurized liquid. In this example, the flexible supply conduits 200 house a supply line and a separate return line, and the misting apparatus are each provided with a return pump 180 to return re-captured liquid along the return line under pressure (not shown).

It will be appreciated that the generator of the portable device may be powered by any suitable power source, for example a diesel generator or electrical generator. Further, it will be appreciated that the misting apparatus may be coupled to other equipment configured to provide pressurized liquid, for example a non-portable source.

Whilst a particular example has been described in which radial arms of a rotary jet assembly have a radial extent such that jet nozzles are located within an annular channel of the jet containment, it will be appreciated that in other examples there may be alternative configurations. For example, no annular channel may be provided in the jet containment. Further, in other examples, the jet nozzles may be positioned radially outward of the tips of the fan, or radially outward of other features of the misting apparatus which define the radial extent of the airflow therethrough, for example features of the housing such as the airflow nozzle or a neck of the air intake. In still further embodiments, the radial arms may be configured so that the jet nozzles are disposed within the radial extent of the airflow, for example behind the disc of the fan, though such arrangements may encourage jets from the jet nozzles to become entrained in the airflow.

Whilst examples have been described in which the airflow nozzle tapers inwardly along the airflow direction, it will be appreciated that in other examples, the nozzle may be un-tapered or may taper outwardly. Un-tapered or outwardly tapering nozzles may promote a wide dispersion of a mist, and may be suitable for a wide range of applications, for example odour control. In at least some such examples, the jet containment may extend radially outwardly of the junction between the air nozzle and the jet containment (i.e. radially outwardly of the proximal end of the airflow nozzle), which may inhibit liquid ejected in the jet containment from entraining in the airflow flowing through the apparatus and being discharged through the airflow nozzle.

## Claims

1. A misting apparatus (100) for producing a mist from a pressurized liquid and ejecting the mist, comprising:
a fan shaft (134) having a rotational axis (104);
a rotary jet assembly (130) configured to drive the fan shaft (134) and comprising a radially extending arm (136) configured to convey the pressurized liquid to a jet nozzle (138), wherein the jet nozzle is configured to eject the pressurized liquid in a jet to generate a jet reaction force having a tangential component;
a fan (140) coupled to the fan shaft for driving an airflow;
a spray nozzle (152) configured to eject the pressurized liquid into the airflow to produce a mist;
a housing (102) comprising a jet containment disposed around the rotary jet assembly for capturing liquid ejected from the jet nozzle; and
a collector (118) for collecting liquid captured by the jet containment and configured to recirculate the liquid to a pump or reservoir.

2. A misting apparatus according to claim 1, wherein the fan shaft comprises a fluid pathway for providing the liquid to the rotary jet assembly; and/or a fluid pathway for providing the liquid to the spray nozzle.

3. A misting apparatus according to any preceding claim, wherein the jet assembly comprises a manifold configured to receive the pressurized liquid and comprising:
a first outlet configured to provide the liquid to a conduit extending along the radial arm to the jet nozzle; and
a second outlet comprising the spray nozzle or configured to provide liquid to the spray nozzle.

4. A misting apparatus according to any preceding claim, wherein the misting apparatus is for receiving a pressurized liquid of variable pressure; and wherein the jet nozzle and the spray nozzle are fluidically coupled so that the flow rate of liquid through the jet nozzle is approximately proportional to the flow rate of liquid through the spray nozzle over a pressure range.

5. A misting apparatus according to any preceding claim, wherein the spray nozzle is mounted to rotate together with the fan shaft in use.

6. A misting apparatus according to any preceding claim, wherein the spray nozzle is detachably attached to the fan shaft.

7. A misting apparatus according to any preceding claim, wherein the fan comprises a plurality of blades each having a radially outer tip; and wherein the jet nozzle is disposed radially outward of the tips of the blades.

8. A misting apparatus according to any preceding claim, further comprising a drive unit coupled to the fan shaft so as to be driven to rotate by rotation of the fan shaft, wherein the drive unit is for driving a liquid return pump for pumping liquid from the collector.

9. A misting apparatus according to claim 8, wherein the misting apparatus comprises:
an attachment point for detachably attaching the liquid return pump so that in use the liquid return pump attached to the attachment point is driven by the drive unit; or
the liquid return pump coupled with the drive unit and arranged to pump liquid from the collector.

10. A misting apparatus according to any preceding claim, wherein the jet nozzle is configured to eject the pressurized liquid along a direction having an axial component.

11. A misting apparatus according to any preceding claim, wherein the housing includes an airflow nozzle, and wherein the apparatus further comprises a drip collector coupled to a downstream end of the airflow nozzle, the drip collector having a radially inner wall defining an outlet of the apparatus radially within the downstream end of the airflow nozzle, a radially outer wall disposed around the downstream end of the airflow nozzle, and a channel therebetween for collecting drips conveyed along the wall of the airflow nozzle.

12. A misting apparatus according to any preceding claim, further comprising a composite line for delivery of pressurized liquid from a source of pressurized liquid and for return of liquid captured from the jet nozzle to the source of pressurized liquid, optionally wherein composite line is flexible.

13. An apparatus, comprising:
a portable device for pressurizing liquid;
one or more misting apparatus in accordance with any preceding claim fluidically coupled to the portable device to receive pressurized liquid;
optionally wherein the misting apparatus is mounted above the portable device on a support.

14. An apparatus according to claim 13, wherein the portable device is configured to pressurize liquid to between 30-200bar; and/or to provide pressurized liquid at a flow rate of between 5-60 litres per minute.

15. A method of suppressing dust in a hazardous environment or controlling odour, the method comprising:
providing an apparatus in accordance with any of claims 1-14 with pressurized liquid between 30-200bar at a flow rate of between 5-60 litres per minute to eject a mist to suppress dust or control odour.

## Patentansprüche

1. Vernebelungsvorrichtung (100) zum Erzeugen eines Nebels aus einer druckbeaufschlagten Flüssigkeit und Ausstoßen des Nebels, die Folgendes umfasst:
eine Ventilatorwelle (134) mit einer Drehachse (104);
eine rotierende Strahlanordnung (130), ausgelegt zum Antreiben der Ventilatorwelle (134) und umfassend einen sich radial erstreckenden Arm (136), ausgelegt zum Fördern der druckbeaufschlagten Flüssigkeit zu einer Strahldüse (138), wobei die Strahldüse ausgelegt ist zum Ausstoßen der druckbeaufschlagten Flüssigkeit in einem Strahl zum Erzeugen einer Strahlreaktionskraft, die eine tangentiale Komponente aufweist;
einen Ventilator (140), gekoppelt mit der Ventilatorwelle, zum Antreiben eines Luftstroms;
eine Sprühdüse (152), ausgelegt zum Ausstoßen der druckbeaufschlagten Flüssigkeit in den Luftstrom zum Erzeugen eines Nebels;
ein Gehäuse (102), umfassend eine Strahleinschließung, angeordnet rund um die rotierende Strahlanordnung zum Aufnehmen von Flüssigkeit, die von der Strahldüse ausgestoßen wurde; und
einen Sammler (118) zum Sammeln von Flüssigkeit, die durch die Strahleinschließung aufgenommen wurde, und ausgelegt zum Zurückführen der Flüssigkeit zu einer Pumpe oder zu einem Behälter.

2. Vernebelungsvorrichtung nach Anspruch 1, wobei die Ventilatorwelle einen Fluiddurchgang zum Bereitstellen der Flüssigkeit für die rotierende Strahlanordnung; und/oder einen Fluiddurchgang zum Bereitstellen der Flüssigkeit für die Sprühdüse umfasst.

3. Vernebelungsvorrichtung nach einem der vorhergehenden Ansprüche, wobei die Strahlanordnung einen Sammler umfasst, der ausgelegt ist zum Aufnehmen der druckbeaufschlagten Flüssigkeit und Folgendes umfasst:
einen ersten Auslass, ausgelegt zum Bereitstellen der Flüssigkeit für eine Leitung, die sich entlang des radialen Arms zur Strahldüse erstreckt; und
einen zweiten Auslass, umfassend die Sprühdüse oder ausgelegt zum Bereitstellen von Flüssigkeit für die Sprühdüse.

4. Vernebelungsvorrichtung nach einem der vorhergehenden Ansprüche, wobei die Vernebelungsvorrichtung dient zum Aufnehmen einer druckbeaufschlagten Flüssigkeit eines variablen Drucks: und wobei die Strahldüse und die Sprühdüse fluidisch gekoppelt sind, sodass die Flussrate von Flüssigkeit durch die Strahldüse über einen Druckbereich annähernd proportional zur Flussrate von Flüssigkeit durch die Sprühdüse ist.

5. Vernebelungsvorrichtung nach einem der vorhergehenden Ansprüche, wobei die Sprühdüse montiert ist, um sich bei Verwendung mit der Ventilatorwelle zu drehen.

6. Vernebelungsvorrichtung nach einem der vorhergehenden Ansprüche, wobei die Sprühdüse lösbar an der Ventilatorwelle befestigt ist.

7. Vernebelungsvorrichtung nach einem der vorhergehenden Ansprüche, wobei der Ventilator mehrere Schaufeln umfasst, die jeweils eine radial auswärtige Spitze aufweisen; und wobei die Strahldüse radial auswärts der Spitzen der Schaufeln angeordnet ist.

8. Vernebelungsvorrichtung nach einem der vorhergehenden Ansprüche, ferner umfassend eine Antriebseinheit, die mit der Ventilatorwelle gekoppelt ist, um durch Drehung der Ventilatorwelle zum Drehen angetrieben zu werden, wobei die Antriebseinheit dient zum Antreiben einer Flüssigkeitsrückleitungspumpe zum Pumpen von Flüssigkeit vom Sammler.

9. Vernebelungsvorrichtung nach Anspruch 8, wobei die Vernebelungsvorrichtung Folgendes umfasst:
einen Befestigungspunkt zum lösbaren Befestigen der Flüssigkeitsrückleitungspumpe, sodass die am Befestigungspunkt befestigte Flüssigkeitsrückleitungspumpe im Einsatz durch die Antriebseinheit angetrieben wird; oder
wobei die Flüssigkeitsrückleitungspumpe mit der Antriebseinheit gekoppelt ist und angeordnet ist, um Flüssigkeit vom Sammler zu pumpen.

10. Vernebelungsvorrichtung nach einem der vorhergehenden Ansprüche, wobei die Strahldüse ausgelegt ist zum Ausstoßen der druckbeaufschlagten Flüssigkeit entlang einer Richtung, die eine axiale Komponente aufweist.

11. Vernebelungsvorrichtung nach einem der vorhergehenden Ansprüche, wobei das Gehäuse eine Luftstromdüse umfasst, und wobei die Vorrichtung ferner einen Tropfensammler umfasst, der mit einem stromabwärtigen Ende der Luftstromdüse gekoppelt ist, wobei der Tropfensammler eine radial innere Wand, die einen Auslass der Vorrichtung radial innerhalb des stromabwärtigen Endes der Luftstromdüse definiert, eine radial äußere Wand, die rund um das stromabwärtige Ende der Luftstromdüse angeordnet ist, und einen Kanal dazwischen zum Sammeln von Tropfen, die entlang der Wand der Luftstromdüse befördert werden, aufweist.

12. Vernebelungsvorrichtung nach einem der vorhergehenden Ansprüche, ferner umfassend eine Verbundwerkstoffleitung zur Lieferung von druckbeaufschlagter Flüssigkeit aus einer Quelle von druckbeaufschlagter Flüssigkeit und zur Rückleitung von Flüssigkeit, die aus der Strahldüse aufgenommen wurde, zur Quelle der druckbeaufschlagten Flüssigkeit, wobei optional die Verbundwerkstoffleitung flexibel ist.

13. Vorrichtung, die Folgendes umfasst:
eine tragbare Vorrichtung zur Druckbeaufschlagung von Flüssigkeit;
eine oder mehrere Vernebelungsvorrichtungen nach einem der vorhergehenden Ansprüche, fluidisch mit der tragbaren Vorrichtung gekoppelt, um druckbeaufschlagte Flüssigkeit aufzunehmen, wobei optional die Vernebelungsvorrichtung über der tragbaren Vorrichtung auf einer Stütze montiert ist.

14. Vorrichtung nach Anspruch 13, wobei die tragbare Vorrichtung ausgelegt ist zum Druckbeaufschlagen von Flüssigkeit auf zwischen 30-200 bar; und/oder zum Bereitstellen von druckbeaufschlagter Flüssigkeit bei einer Flussrate von zwischen 5-60 Litern pro Minute.

15. Verfahren zum Unterdrücken von Staub in einer gefährlichen Umgebung oder zum Steuern von Geruch, wobei das Verfahren Folgendes umfasst:
Bereitstellen einer Vorrichtung nach einem der Ansprüche 1-14 mit druckbeaufschlagter Flüssigkeit zwischen 30-200 bar bei einer Flussrate von zwischen 5-60 Litern pro Minute zum Ausstoßen eines Nebels zum Unterdrücken von Staub oder Steuern von Geruch.

## Revendications

1. Appareil de pulvérisation (100) destiné à produire un brouillard à partir d'un liquide sous pression et à éjecter le brouillard, comprenant :
un arbre de ventilateur (134) ayant un axe de rotation (104) ;
un ensemble de jet rotatif (130) conçu pour entraîner l'arbre de ventilateur (134) et comprenant un bras s'étendant radialement (136) conçu pour transporter le liquide sous pression vers une buse de jet (138), la buse de jet étant conçue pour éjecter le liquide sous pression en un jet pour générer une force de réaction de jet ayant une composante tangentielle ;
un ventilateur (140) accouplé à l'arbre de ventilateur pour entraîner un courant d'air ;
une buse de pulvérisation (152) conçue pour éjecter le liquide sous pression dans le courant d'air afin de produire un brouillard ;
un boîtier (102) comprenant une enceinte de confinement de jet disposée autour de l'ensemble de jet rotatif pour capturer le liquide éjecté par la buse de jet ; et
un collecteur (118) pour collecter le liquide capturé par l'enceinte de confinement de jet et conçu pour remettre en circulation le liquide vers une pompe ou un réservoir.

2. Appareil de pulvérisation selon la revendication 1, l'arbre de ventilateur comprenant un chemin de fluide pour fournir le liquide à l'ensemble de jet rotatif ; et/ou un chemin de fluide pour fournir le liquide à la buse de pulvérisation.

3. Appareil de pulvérisation selon l'une quelconque des revendications précédentes, l'ensemble de jet comprenant un manifold conçu pour recevoir le liquide sous pression et comprenant :
une première sortie conçue pour fournir le liquide à un conduit s'étendant le long du bras radial jusqu'à la buse de jet ; et
une seconde sortie comprenant la buse de pulvérisation ou conçue pour fournir du liquide à la buse de pulvérisation.

4. Appareil de pulvérisation selon l'une quelconque des revendications précédentes, l'appareil de pulvérisation étant destiné à recevoir un liquide sous pression de pression variable ; et la buse de jet et la buse de pulvérisation étant accouplées fluidiquement de sorte que le débit de liquide à travers la buse de jet soit approximativement proportionnel au débit de liquide à travers la buse de pulvérisation sur une plage de pression.

5. Appareil de pulvérisation selon l'une quelconque des revendications précédentes, la buse de pulvérisation étant montée pour tourner avec l'arbre de ventilateur lors de l'utilisation.

6. Appareil de pulvérisation selon l'une quelconque des revendications précédentes, la buse de pulvérisation étant attachée de manière amovible à l'arbre de ventilateur.

7. Appareil de pulvérisation selon l'une quelconque des revendications précédentes, le ventilateur comprenant une pluralité de pales ayant chacune une extrémité radialement extérieure ; et la buse de jet étant disposée radialement vers l'extérieur des extrémités des pales.

8. Appareil de pulvérisation selon l'une quelconque des revendications précédentes, comprenant en outre une unité d'entraînement accouplée à l'arbre de ventilateur de sorte à être entraînée en rotation par la rotation de l'arbre de ventilateur, l'unité d'entraînement étant destinée à entraîner une pompe de retour de liquide pour pomper le liquide du collecteur.

9. Appareil de pulvérisation selon la revendication 8, l'appareil de pulvérisation comprenant :
un point d'attache pour attacher de manière amovible la pompe de retour de liquide de sorte que lors de l'utilisation, la pompe de retour de liquide attachée au point d'attache soit entraînée par l'unité d'entraînement ; ou
la pompe de retour de liquide accouplée à l'unité d'entraînement et disposée pour pomper le liquide du collecteur.

10. Appareil de pulvérisation selon l'une quelconque des revendications précédentes, la buse de jet étant conçue pour éjecter le liquide sous pression le long d'une direction ayant une composante axiale.

11. Appareil de pulvérisation selon l'une quelconque des revendications précédentes, le boîtier comprenant une buse de courant d'air, et l'appareil comprenant en outre un collecteur de gouttes accouplé à une extrémité aval de la buse de courant d'air, le collecteur de gouttes ayant une paroi radialement intérieure définissant une sortie de l'appareil radialement à l'intérieur de l'extrémité aval de la buse de courant d'air, une paroi radialement extérieure disposée autour de l'extrémité aval de la buse de courant d'air, et un canal entre elles pour collecter les gouttes transportées le long de la paroi de la buse de courant d'air.

12. Appareil de pulvérisation selon l'une quelconque des revendications précédentes, comprenant en outre une ligne composite pour la fourniture d'un liquide sous pression à partir d'une source de liquide sous pression et pour le retour du liquide capturé de la buse à jet vers la source de liquide sous pression, éventuellement la ligne composite étant flexible.

13. Appareil, comprenant :
un dispositif portable pour mettre sous pression un liquide ;
au moins un appareil de pulvérisation selon l'une quelconque des revendications précédentes accouplé fluidiquement au dispositif portable pour recevoir un liquide sous pression ;
éventuellement l'appareil de pulvérisation étant monté au-dessus du dispositif portable sur un support.

14. Appareil selon la revendication 13, le dispositif portable étant conçu pour mettre sous pression un liquide à une pression comprise entre 30 et 200 bars ; et/ou pour fournir un liquide sous pression à un débit compris entre 5 et 60 litres par minute.

15. Procédé de suppression de la poussière dans un environnement dangereux ou de contrôle des odeurs, le procédé comprenant l'étape consistant à :
fournir un appareil selon l'une quelconque des revendications 1 à 14 avec un liquide sous pression entre 30 et 200 bars à un débit compris entre 5 et 60 litres par minute pour éjecter un brouillard afin de supprimer la poussière ou de contrôler les odeurs.
